Europäisches Patentamt

European Patent Office

Office européen des brevets

(11) Publication number: **0 413 053 A1**

# EUROPEAN PATENT APPLICATION

(21) Application number: 89115280.3

(51) Int. Cl.5: **A61K 35/84**

(22) Date of filing: 18.08.89

(43) Date of publication of application:
20.02.91 Bulletin 91/08

(84) Designated Contracting States:
AT BE CH DE ES FR GB GR IT LI LU NL SE

(71) Applicant: **NIPPON HYPOX LABORATORIES INCORPORATED**
1759, Matsugaya Hachioji-shi
Tokyo(JP)

(72) Inventor: **Satoh, Toshio**
**57-3, Nagao Jyoroku-cho**
**Tokushima-shi Tokushima-ken(JP)**
Inventor: **Niiro, Yasunori No. 403, 2nd**
**Masuoka Bldg.**
**1-3, Minamisako-hachiban-cho**
**Tokushima-shi Tokushima-ken(JP)**
Inventor: **Kakegawa, Hisao No. 307,**
**City-Corpo**
**Kimura 3-59, Okihama-higashi**
**Tokushima-shi Tokushima-ken(JP)**
Inventor: **Ishii, Hiroshi 102, Aza-Minamihiraki**
**Higashi-Umazume Oasa-cho**
**Naruto-shi Tokushima-ken(JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) Process for producing useful substance from edible basidiomycete mycelium.

(57) The present invention relates to a useful substance obtained by subjecting a mycelium of a particular edible basidiomycete as a starting material to a particular culture operation and a particular extraction operation. This substance has an anti-allergic action and is useful as a drug.

EP 0 413 053 A1

# PROCESS FOR PRODUCING USEFUL SUBSTANCE FROM EDIBLE BASIDIOMYCETE MYCELIUM

## BACKGROUND OF THE INVENTION

### (1) Field of the Invention

The present invention relates to a process for producing a useful substance from an edible basidiomycete mycelium. The substance obtained by the process of the present invention has an anti-allergic action and can be used in drugs, quasi-drugs, cosmetics, foods, etc.

### (2) Description of the Prior Art

In recent years, some of foods has been evaluated not only by their nutrition or taste but also by their physiological activities. Researches on these efficacious foods for health are under way in many fields.

When an excellent physiologically active substance is found in our daily foods, the substance is thought to have a high probability of being used as a drug, because the safety of the substance is already proven. There is a literature describing that many basidiomycetes such as Lentinula edodes and the like contain antitumor polysaccharides [Chihara, "Protein, Nucleic Acid and Enzyme", 21(6)424 (1976)].

A life history of any basidiomycete is largely divided into (a) a period wherein the basidiomycete exists as a mycelium and (b) a period wherein the basidiomycete grows and multiplicates to form a fruit body or a hymenium. Antitumor polysaccharides are obtained from culture products of mycelia of Coriolus versicolor, Schizophyllum commune, etc.; Lentinan (an antitumor polysaccharide) is found in the fruit body of Lentinula edodes; these antitumor polysaccharides are being used as respective drugs. With respect to Lentinula edodes, there is a literature describing that the culture product of the mycelium has an immunomodulating action and thereby shows an antihepatitis action [Mizoguchi, "Journal of Liver, Gall-bladder and Pancreas" 15, 127 (1987)].

However, there is no case yet in which the anti-allergic action of a culture product of any edible basidiomycete mycelium has been proven scientifically.

## SUMMARY OF THE INVENTION

Hence, the object of the present invention is to obtain from edible mushrooms a substance which is usable as a medicinal material or an efficacious food for health.

The present inventors found that a useful substance having an anti-allergic action can be obtained by culturing a mycelium of an edible basidiomycete selected from Grifola frondosa, Pisolithus tinctorius, Panellus serotinus, Agaricus blazei murrill, Auricularia polytricha and Tremella fuciformis (all having been used for food from of old), in a medium comprising a saccharide containing glucose in the molecule; subjecting the resulting culture liquid to centrifugation or filtration to obtain a solid culture product; extracting the solid culture product with water and/or a hydrophilic organic solvent; and subjecting the resulting extract to concentration and drying.

Therefore, the present invention lies in a process for producing a useful substance from an edible basidomycete mycelium, which process comprises culturing a mycelium of an edible basidiomycete selected from Grifola frondosa, Pisolithus tinctorius,Panellus serotinus, Agaricus blazei murrill, Auricularia polytricha and Tremella fuciformis, in a medium comprising a saccharide containing glucose in the molecule; subjecting the resulting culture liquid to centrifugation or filtration to obtain a solid culture product; extracting the solid culture product with water and/or a hydrophilic organic solvent; and subjecting the resulting extract to concentration and drying to obtain a useful substance.

## DETAILED DESCRIPTION OF THE INVENTION

The present invention is described in detail below.

As the edible basidiomycete usable in the process for producing a useful substance according to the present invention, there can be mentioned Grifola frondosa, Pisolithus tinctorius, Panellus serotinus, Agaricus blazei murrill, Auricularia polytricha and Tremella fuciformis. The mycelium of each of these edible basidiomycetes can be cultured as follows. That is, the mycelium of an edible basidiomycete selected from Grifola frondosa, Pisolithus tinctorius, Panellus serotinus, Agaricus blazei murrill, Auricularia polytricha and Tremella fuciformis is separated and then cultured using a medium comprising a saccharide containing glucose in the molecule.

In the above medium, as the saccharide as a carbon source containing glucose in the molecule, there can be mentioned glucose, maltose, trehalose, sucrose, starch, etc. Preferably, the medium also contains a nitrogen source. Preferable as the nitrogen source are organic nitrogen coumpounds such as Casamino acid (a mixture of amino acids) and the like, as well as natural organic products such as tomato, bamboo shoot, potato and the like. From these viewpoints, the medium used in the present invention is preferably a tomato medium to which a saccharide containing glucose in the molecule has been added, a Casamino acid-containing synthetic medium to which a saccharide containing glucose in the molecule has been added, a bamboo shoot medium which per se contains a saccharide containing glucose in the molecule, or a potato medium which per se contains a saccharide containing glucose in the molecule. When there is used a minimum essential medium, for example, a glutamic acid medium or a medium using, as a nitrogen source, only an ammonium salt (e.g. ammonium chloride) or a nitrate (e.g. ammonium nitrate, sodium nitrate, potassium nitrate), multiplication of mycelium is very slow.

When there is used a medium comprising a saccharide containing no glucose in the molecule, for example, a medium using, as a carbon source, only xylose, fructose or the like, multiplication of mycelium takes place but there can be obtained no culture product having an anti-allergic action. The physical conditions of culture can be those ordinarily used in culture and have no particular restriction. However, the culture temperature is 15-40°C, preferably 20-30°C. The culture is preferably effected under a light-shielded dark condition, but may be allowed under light application of 11-14 hours per day. The pH of culture is preferably 3.0-8.0, more preferably 3.5-7.0.

With respect to the stirring during culture, several minutes (per every 24 hours) of reciprocating shaking or rotary shaking is sufficient, but continuous shaking may also be employed.

The culture period varies depending upon the physical conditions of culture and the composition of medium, but is preferably 3-120 days, particularly preferably 5-90 days.

After the culture, the culture liquid is subjected to centrifugation or filtration to obtain a solid culture product. The centrifugation is effected by an centrifugal operation giving a gravitational acceleration of 100-5,000 g, preferably 800-3,000 g. The filtration is effected using a sieve of 3.5-200 meshes, particularly preferably 4-16 meshes, a filter paper, a gauze or the like.

The thus obtained solid culture product is extracted with a solvent, i.e. water and/or a hydrophilic solvent (e.g. methanol, ethanol, acetone, tetrahydrofuran). The temperature during extraction is 4-120°C, preferably 40-90°C. It is preferable that the solid culture product be ground or cut into very small pieces before extraction. The extraction solvent is used in an amount of 1-100 parts by weight, preferably 3-20 parts by weight per 1 part by weight (as wet) of the solid product. The extraction time is 1-48 hours, preferably 6-24 hours.

The thus obtained extract is optionally combined with the supernatant of the above centrifugation or the filtrate of the above filtration and then concentrated. The means for concentration has no particular restriction, but concentration under vacuum or freeze drying is advantageously used in order to prevent the denaturation of desired substance, etc.

The useful substance of the present invention obtained after the concentration is an yellowish white to brown hygroscopic solid substance and has been confirmed by the phenol-sulfuric acid method or the anthrone method to contain not only saccharides but also fatty acids, sterols, etc.

As is clear from Examples which are described later, the useful substance of the present invention has an inhibitory action against the reaction of histamine liberation from mast cells which is one cause of allergic reaction in vivo and, moreover, has a high safety. Therefore, the substance is useful as a drug for preventing or curing various allergic diseases, for example, atopic dermatitis, allergic nastis and bronchial asthma. The substance also exhibits the action by being incorporated into cosmetics, quasi-drugs, foods, etc.

The present invention is described in more detail below by way of Examples.

(1) Example of production of useful substances from edible basidiomycete mycelia

(a) Preparation of media

Each medium used in culture was prepared as follows.

(Tomato medium)

200 g of slices of tomato sarcocarp were added to 1,000 ml of distilled water, and the mixture was heated on a boiling water bath for 1 hour. When hot, the mixture was filtered to remove the solid portion. In the filtrate was dissolved 20 g of D-glucose, and the solution was adjusted to pH 6.0 if necessary. The resulting solution was subjected to autoclaving (120°C x 20 minutes) to obtain a tomato medium.

(Potato medium)

Potato tuber was peeled and then made into slices. 200 g of the slices were added to 1,000 ml of distilled water. The mixutre was heated on a boiling water bath for 1 hour. The subsequent procedure was the same as in the preparation of tomato medium, to obtain a potato medium.

(Bamboo shoot medium)

The edible portion of bamboo shoot was made into slices. 200 g of the slices were added to 1,000 ml of distilled water. The mixture was heated on a boiling water bath for 1 hour. The subsequent procedure was the same as in the preparation of tomato medium, to obtain a bamboo shoot medium.

| (Casamino acid-containing synthetic medium) | |
| --- | --- |
| Saccharide* | 20.0 g |
| Casamino acid | 4.04 g |
| Adenine hydrochloride | 5.0 mg |
| Guanine hydrochloride | 5.0 mg |
| Cytosine hydrochloride | 2.0 mg |
| Thymine | 2.0 mg |
| Uracil | 2.0 mg |
| Inositol | 2.5 mg |
| Nicotinamide | 0.2 mg |
| Riboflavin | 0.2 mg |
| Pyridoxine hydrochloride | 0.1 mg |
| Folic acid | 0.1 mg |
| Biotin | 0.1 mg |
| Cyanocobalamin | 0.1 mg |
| Orotic acid | 0.2 mg |
| Thiamine hydrochloride | 0.2 mg |
| Potassium dihydrogenphosphate | 1.0 g |
| Magnesium sulfate | 0.3 g |
| Calcium chloride | 0.1 g |
| Sodium chloride | 0.05 g |
| Zinc sulfate | 0.2 mg |
| Ferrous sulfate | 0.2 mg |
| Copper sulfate | 0.1 mg |
| Manganese sulfate | 0.1 mg |

(* There was used, as the saccharide, one of glucose, maltose, trehalose, starch, xylose and fructose.)

The above compositions each containing one of glucose, maltose, trehalose, starch, xylose and fructose were dissolved in distilled water and the solution was adjusted to pH 6.0. Distilled water was further added to make the total volume to 1,000 ml. The resulting solutions were subjected to autoclaving (120°C x 20 minutes) to obtain six kinds of Casamino acid-containing synthetic media.

(b) Culture of basidiomycetes

A mycelium piece taken out, by cutting, from a colony of a vegetative hypha of Grifola frondosa, Pisolithus tinctorius, Panellus serotinus, Agaricus blazei murrill, Auricularia polytricha and Tremella fuciformis as well as A. rubescens, Volvariella volvacea, Flammulina velutipes, Lentinula edodes and Pleurotus ostreatus was inoculated into a culture bottle into which one of the above obtained media had been aseptically poured, and cultured at 25° C for 1-12 weeks in a dark place.

In Tables 1 and 2 are shown the type of medium used in culture of the mycelium of each basidiomycete.

After culture, the solid culture product was collected by filtration through a gauze and cut into small pieces. The small pieces were mixed with distilled water of 10 times the wet weight of the small pieces, and the mixture was heated on a boiling water bath for 1 hour. The supernatant was separated. The same extraction was repeated. The extract and the supernatant were combined, concentrated under vacuum to a 1/20-fold or less volume, and freeze-dried to obtain a desired substance.

The thus obtained substance was a yellowish white to brown solid and was confirmed to contain saccharides, fatty acids, sterols, etc.

The substance obtained from each mycelium was mixed with methanol and the resulting supernatant was subjected to thin-layer chromatography. The plate was silica gel 60 manufactured by Merck Co.; the developing solvent was chloroform : methanol = 9 : 1. Color development by iodine was used for detection.

The result of thin-layer chromatography of each substance obtained from each basidiomycete is shown below.

Tremella fuciformis

Spot at Rf = 0.44
Tailing from the original point to around Rf = 0.06

Auricularia polytricha

Spots at Rf = 0.25, 0.36, 0.42, 0.46, 0.51, 0.59 and 0.84
Tailing from the original point to around Rf = 0.15

Agaricus blazei murrill

Spots at Rf = 0.51 and 0.84
Tailing from the original point to around Rf = 0.05

Pisolithus tinctorius

Spots at Rf = 0.51 and 0.84
Tailing from the original point to around Rf = 0.05

Panellus serotinus

Spots at Rf = 0.49 and 0.84
Tailing from the original point to around Rf = 0.06

Grifola frondosa

Spots at Rf = 0.53 and 0.84
Tailing from the original point to around Rf = 0.06

(2) Example of pharmacological test of substances obtained by the process of the present invention

(a) The substances obtained using the basidiomycetes and media shown in Table 1 were tested for their actions on the reaction of histamine liberation from rat peritoneal mast cells (this reaction is widely used for the assay of an anti-allergic agent, as a model of allergic reaction taking place in vivo).

5

That is, in a Locke's solution were suspended about $1 \times 10^6$ (per ml of Locke's solution) of rat peritoneal mast cells. Thereto was added 2 g/ml of a test sample, and the mixture was stirred to obtain a uniform solution. The solution was heated at 37°C for 5 minutes. Then, the compound 48/80 was added and the mixture was heated at 37°C for 10 minutes. After ice cooling, the mixture was subjected to centrifugation at 2,500 rpm at 4°C. Both the supernatant and the residue were measured for histamine amount by the method of P. A. Shore (J. Exp. Ther. 127, 182 (1959)) to determine the action on histamine liberation. The results are shown in Table 1. As is clear from Table 1, the substances obtained from Grifola frondosa, Pisolithus tinctorius, Pnellus serotinus, Agaricus blazei murrill, Auricxularia polytricha and Tremella fuciformis all showed a strong inhibitory action for histamine liberation.

Incidentally, the comparative substances obtained from A. rubescens, Volvariella, Flammulina velutipes, etc. showed no inhibitory action at all.

(b) The substances obtained from Tremella fuciformis, using one of the Casamino acid-containing synthetic media, (said media being prepared in (1) (a) and each containing a saccharide selected from glucose, maltose, trehalose, starch, xylose and fructose) were measured for (a) multiplicability and (b) inhibitory action for histamine liberation. In (a) and (b), 1 mg/ml of the substance is used. The results are shown in Table 2. As is clear from Table 2, in all cases of the media, the substances showed an inhibitory action for histamine liberation, but the inhibitory action was strong when the medium contained glucose, maltose, trehalose or starch and weak when the medium contained xylose or fructose. Separately, Tremella fuciformis was cultured in the same manner in media each containing, as a nitrogen source, ammonium nitrate, potassium nitrate, ammonium chloride or peptone in place of Casamino acid and further containing given amounts of glucose, nucleic acid and thiamine, and it was found that ammonium nitrate, potassium nitrate, ammonium chloride or peptone gave slow multiplication and Casamino acid had the advantage over them.

Table 1

| Edible basidiomycete | Medium | Inhibition for histamine liberation (%) |
|---|---|---|
| Grifola frondosa | Tomato medium | 44.1 |
| A. rubescens | Same as above | No inhibition |
| Pisolithus tinctorius | Same as above | 74.7 |
| Panellus serotinus | Same as above | 85.1 |
| Agaricus blazei murrill | Bamboo shoot medium | 67.2 |
| Auricularia polytricha | Tomato medium | 43.8 |
| Tremella fuciformis | Same as above | 93.2 |
| Volvariella volvacea | Tomato medium plus potato medium | No inhibition |
| Flammulina velutipes | Same as above | Same as above |
| Lentinula edodes | Same as above | Same as above |
| Pleurotus ostreatus | Same as above | Same as above |

Table 2

| Edible basidiomycete | Medium | Saccharide in medium | Dry weight of solid culture product obtained by culturing a mycelium for 28 days (g/bottle) | Inhibition for histamine liberation (%) |
|---|---|---|---|---|
| Tremella fuciformis | Casamino acid-containing synthetic medium | Glucose | 0.069 | 92.0 |
| Same as above | Same as above | Maltose | 0.088 | 72.7 |
| Same as above | Same as above | Trehalose | 0.080 | 80.2 |
| Same as above | Same as above | Starch | 0.076 | 83.5 |
| Same as above | Same as above | Xylose | 0.055 | 26.2 |
| Same as above | Same as above | Fructose | 0.101 | 22.0 |

EP 0 413 053 A1

(3) Example of toxicity test of substances obtained from edible basidiomycetes

The substances obtained by the process of the present invention were tested for acute toxicity by oral administration to ddY mice. There was no death at a dose of 10 g/kg in all the substance groups.

As described in detail above, there have been provided, according to the present invention, substances which have an anti-allergic action and a high safety and accordingly are useful as a curing or preventive agent for allergic diseases and further can be incorporated into cosmetics, foods, etc.

## Claims

1. A process for producing a useful substance having an anti-allergic action from an edible basidiomycete mycelium, which process comprises culturing a mycelium of an edible basidiomycete selected from Grifola frondosa, Pisolithus tinctorius, Panellus serotinus, Agaricus blazei murrill, Auricularia polytricha and Tremella fuciformis in a medium comprising a saccharide containing glucose in the molecule; subjecting the resulting culture liquid to centrifugation or filtration to obtain a solid culture product; extracting the solid culture product with water and/or a hydrophilic organic solvent; and subjecting the resulting extract to concentration and drying to obtain a useful substance.

2. A process according to Claim 1, wherein the medium is a tomato medium to which a saccharide containing glucose in the molecule has been added, or a Casamino acid-containing synthetic medium to which a saccharide containing glucose in the molecule has been added.

3. A process according to Claim 1, wherein the medium is a bamboo shoot medium which per se contains a saccharide containing glucose in the molecule, or a potato medium which per se contains a saccharide containing glucose in the molecule.

4. A process according to Claim 1, wherein the culture is effected at pH 3.0-8.0 at 15-40°C for 3-120 days.

5. A process according to Claim 1, wherein the culture is effected under a light-shielded dark condition or under light application for 11-14 hours per day.

6. A process according to Claim 1, wherein the extraction is effected at 4-120°C using 1-100 parts by weight, based on the wet weight of the solid culture product, of water and/or the hydrophilic organic solvent.

7. A process according to Claim 1, wherein the hydrophilic organic solvent is at least one member selected from methanol, ethanol, acetone and tetrahydrofuran.

8. A useful substance having an anti-allergic action, which is obtained by a process according to Claim 1.

9. A composition containing, as an active ingredient, a useful substance according to Claim 8, which can be used in at least one application selected from the group consisting of drugs, quasi-drugs, cosmetics and foods.

8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN, vol. 8, no. 77 (C-218)[1514], 10th April 1984; & JP-A-59 1427 (NODA SHIYOTSUKIN K.K.) 06-01-1984 * Whole abstract * | 1-9 | A 61 K 35/84 |
| A | PATENT ABSTRACTS OF JAPAN, vol. 12, no. 70 (C-479)[2917], 4th March 1988; & JP-A-62 209 023 (SUNSTAR INC.) 14-09-1987 | | |
| A | PATENT ABSTRACTS OF JAPAN, vol. 13, no. 134 (C-581)[3482], 4th April 1989; & JP-A-63 297 400 (FUJIMOTO SEIYAKU K.K.) 05-12-1988 | | |
| A | FR-A-2 335 235 (KUREHA KAGAKU K.K.) | | |
| A | PATENT ABSTRACTS OF JAPAN, vol. 12, no. 450 (C-547)[3297], 25th November 1988; & JP-A-63 173 583 (AKIRA YAMAZAKI) | | TECHNICAL FIELDS SEARCHED (Int. Cl.5) A 61 K C 12 N |

The present search report has been drawn up for all claims

| Place of search | Date of completion of the search | Examiner |
|---|---|---|
| THE HAGUE | 02-04-1990 | ALVAREZ Y ALVAREZ C. |